# EUROPÄISCHE PATENTANMELDUNG

(11) **EP 0 970 943 A2**
(43) Veröffentlichungstag der Anmeldung: **12.01.2000**
(21) Anmeldenummer: 99110655.0
(22) Anmeldetag: 02.06.1999
(51) Int. Cl.: C07C 209/58

(54) **Verfahren zur Herstellung von Cyclopropylamin**

(30) Priorität: 09.07.1998 DE 19830633
(71) Anmelder: Degussa-Hüls Aktiengesellschaft, 60287 Frankfurt am Main (DE)
(72) Erfinder: Kaufhold, Manfred Dr., 45770 Marl (DE); Kleemiss, Wolfgang Dr., 45721 Haltern (DE); Feld, Marcel Dr., 51147 Köln (DE)

(57) **Zusammenfassung**

Die Erfindung betrifft ein Verfahren zur Herstellung von Cyclopropylamin, ausgehend von Butyrolacton, bei dem man (a) Butyrolacton mit Chlorwasserstoff zu Chlorbuttersäure umsetzt, (b) die Chlorbuttersäure mit einem primären oder sekundären Alkohol mit 4 bis 8 Kohlenstoffatomen verestert, (c) den Ester mit einem Alkalialkoholat eines primären Alkohols mit 1 bis 3 Kohlenstoffatomen zu einem Gemisch von Cyclopropancarbonsäureestern cyclisiert, (d) das Cyclopropancarbonsäureester-Gemisch durch Umsetzung mit Ammoniak in Cyclopropancarbonsäureamid umwandelt und (e) das Cyclopropancarbonsäureamid durch Hofmannschen Abbau in Cyclopropylamin überführt.

## Beschreibung

Die Erfindung betrifft ein Verfahren zur Herstellung von Cyclopropylamin aus gamma-Butyrolacton (in der Folge kurz als "Butyrolacton" bezeichnet). Die Erfindung betrifft insbesondere die Stufe des Verfahrens, in der ein gamma-Chlorbuttersäureester ("Chlorbuttersäureester") zu einem Cyclopropancarbonsäureester cyclisiert wird.

Cyclopropylamin ist ein wichtiges Vorprodukt für Pharmaka und Agrochemikalien. Ein kostengünstiges Herstellverfahren ist daher erwünscht. Üblicherweise stellt man Cyclopropylamin aus gamma-Butyrolacton über die Zwischenstufen gamma-Chlorbuttersäure ("Chlorbuttersäure"). Chlorbuttersäureester, Cyclopropancarbonsäureester und Cyclopropancarbonsäureamid her. Diese Reaktionskette sowie einzelne Zwischenstufen sind mehrfach beschrieben worden. So ist bekannt, daß Cyclopropancarbonsäureethylester durch Cyclisierung von Chlorbuttersäureethylester mit Natrium-t-amylat als Ringschlußbase hergestellt werden können, wobei die Ausbeute nur 45% betrug (Julia et al. , Bull.Soc.Chim.France **1960**. 306 ff.). Ebenfalls unbefriedigend ist die Ausbeute von 66%, die bei der Herstellung von Cyclopropancarbonsäureethylester durch Reaktion von Chlorbuttersäureethylester mit Natriummethylat erreicht wurde (Bunce et al . , Organic Preparations and Procedures **6**, 193-6 [1969]).

Die erwähnte fünfstufige Reaktionsfolge zur Herstellung von Cyclopropylamin in Form einer wäßrigen Lösung wird z.B. in US-A 3,711,549 oder DE-OS 19 39 759 beschrieben. Dabei wird zunächst Butyrolacton mit Chlorwasserstoff in Chlorbuttersäure überführt, die anschließend oder gleichzeitig mit einem niederen Alkanol zum Chlorbuttersäurealkylester verestert wird. Dieser wird in Gegenwart eines inerten Lösemittels, wie Toluol, zum Cyclopropancarbonsäureester cyclisiert, der dann im Reaktionsgemisch mit Ammoniak unter Basen-Katalyse zum Cylopropancarbonsäureamid reagiert, das schließlich in einer Hofmann-Reaktion zum Cyclopropylamin abgebaut wird. In den Schriften werden als Chlorbuttersäureester lediglich der Methylester und als Base zum Ringschluß lediglich Natriummethylat erwähnt. Die Gesamtausbeute an Cyclopropylamin, bezogen auf Chlorbuttersäureester, wird mit 80% angegeben, die Ausbeute im Cyclisierungsschritt mit etwa 92%. Bei diesem Verfahren ist nachteilig, daß niedere Ester der Chlorbuttersäure schwieriger herzustellen sind als die Ester höherer Alkohole. Dies liegt daran, daß das bei der Reaktion entstehende Wasser nur mit höheren Alkoholen aus dem Reaktionsgemisch azeotrop ausgetragen und im Kondensat als gesonderte Phase abgetrennt werden kann. Der Zusatz eines inerten, mit Wasser nicht mischbaren Lösemittels zur azeotropen Entfernung von Wasser führt zu erhöhtem Destillationsaufwand.

Einen ähnlichen Nachteil weist auch das Verfahren nach EP-A1 0 043 949 auf, das zwei Zwischenstufen zusammenfaßt. Dabei wird Chlorbuttersäuremethyl- oder -ethylester mit Natrium- oder Kaliummethylat in Gegenwart von flüssigem Ammoniak in einem Schritt zum Cyclopropancarbonsäuremethyl- oder -ethylester cyclisiert und dieser amidiert. Die Ausbeute an Cyclopropancarbonsäureamid beträgt zwar >90%. Flüssiges Ammoniak ist jedoch ein Stoff, dessen Verwendung besondere Sicherheitsmaßnahmen erfordert. Außerdem ist die Dosierung von flüssigem Ammoniak apparatetechnisch aufwendiger als die Dosierung von Ammoniak in Gasform.

Nach EP-B1 0 205 403 cyclisiert man in einem von Butyrolacton ausgehenden Verfahren zur Herstellung von Cyclopropylamin Chlorbuttersäureester sekundärer oder tertiärer Alkohole zu einem sterisch gehinderten Cyclopropancarbonsäureester mittels festen Natriumhydroxids in einem inerten Lösemittel unter Zusatz eines Phasentransferkatalysators. Ester tertiärer Alkohole, wie tert. -Butanol , ergaben die besten Resultate. Von den Estern sekundärer Alkohole wurden der Isopropyl- und der 2-Butylester genannt. Die Ausbeuten werden mit >90% angegeben. Dabei muß jedoch der Chlorbuttersäureester durch Destillation gereinigt werden, was das Verfahren aufwendig macht, ebenso wie die Verwendung von Phasentransferkatalysatoren, wie Tributylmethylammoniumchlorid. Zudem bedarf der nachfolgende Amidierungsschritt des relativ aufwendig herzustellenden Mononatriumethylenglykolats. Die Ausbeute an Cyclopropylamin über alle Stufen beträgt etwa 80%.

Auch die Zwischenstufe, in der ein Cyclopropancarbonsäureester zum Amid umgesetzt wird, ist bereits mehrfach beschrieben worden. So werden gemäß EP-B1 0 662 470 Cyclopropancarbonsäureester niederer Alkohole mit 1 bis 3 Kohlenstoffatomen unter Verwendung eines Alkalialkoholats eines einwertigen Alkohals mit 1 bis 8 Kohlenstoffatomen als Katalysator amidiert. Hierbei ist kein inertes Lösemittel erforderlich, um gute Ausbeuten zu erzielen. Die Reaktion wird jedoch nur bis zu einem Esterumsatz von 60 bis 90% geführt, damit das Reaktionsgemisch handhabbar bleibt. Nach der Abtrennung des Cyclopropancarbonsäureamids kann die Mutterlauge, die noch Cyclopropancarbonsäureester enthält, erneut der Amidierung zugeführt werden, so daß schließlich Ausbeuten von >90% erzielt werden. Dieses Verfahren ist nur dann vorteilhaft, wenn Cyclopropancarbonsäureester niederer Alkohole mit 1 bis 3 Kohlenstoffatomen günstig zur Verfügung stehen.

Das Verfahren nach EP-B1 0 365 970 führt ebenfalls vom Cyclopropancarbonsäureester zum Cyclopropancarbonsäureamid. Man setzt einen Ester eines C₄- bis C₈-Alkohols ein, verwendet ein Alkalialkoholat eines C₁- bis C₈-Alkohols und arbeitet mit gasförmigem Ammoniak. Dabei liegen die Ausbeuten >95%. Ein besonders bevorzugte Ester ist der Isopropylester, über dessen Herstellung keine Angaben gemacht werden. Bei dem Verfahren ist die Raum-Zeit-Ausbeute gering, da die Cyclopropancarbonsäureester höherer Alkohole ein relativ hohes Molekulargewicht haben, während Cyclopropancarbonsäureamid ein vergleichsweise niedriges Molekulargewicht besitzt.

Der Hofmannsche Abbau von Cyclopropancarbonsäureamid zum Cyclopropylamin ist in drei weiteren Patentschriften beschrieben. Man kann das Amid einerseits in Lösung entweder in einem Temperaturbereich von 10 bis 35°C (EP-B1 0 367 010) oder von 45 bis 260°C (AP-A1 0 393 350) kontinuierlich zum Amin abbauen. Andererseits gelingt der Abbau halbkontinuierlich in Suspension, wenn man nach der Vorchlorierung des Amids, die bei niedriger Temperatur abläuft, die dann homogene Lösung kontinuierlich bei erhöhter Temperatur durch einen Rohrreaktor führt. (DE 195 23 868.0). In allen Fällen erhält man Ausbeuten an Cyclopropylamin von >90%, bezogen auf das eingesetzte Amid. Die genannten Verfahren setzen die Verfügbarkeit von reinem Cyclopropancarbonsäureamid voraus, das auf den zuvor geschilderten recht aufwendigen Wegen hergestellt werden muß.

Trotz der zahlreichen Vorschläge bestand weiterhin ein Bedarf an einem verbesserten Verfahren zur Herstellung von Cyclopropylamin. ausgehend von Butyrolacton, das eine hohe Gesamtausbeute und hohe Raum-Zeit-Ausbeuten ergibt, mit leicht zugänglichen Katalysatoren arbeitet und ohne inerte Lösemittel auskommt. Es bestand weiterhin Bedarf an einer verbesserten Cyclisierungsreaktion, die in dem genannten Verfahren verwendet werden kann und von leicht verfügbaren Chlorbuttersäureestern ausgeht. Es war eine Aufgabe der Erfindung, ein solches verbessertes Verfahren und eine solche verbesserte Cyclisierungsreaktion bereitzustellen.

Es wurde nun gefunden, daß sich Cyclopropylamin, ausgehend von Butyrolacton, vorteilaft herstellen läßt, indem man
(a) Butyrolacton mit Chlorwasserstoff zu Chlorbuttersäure umsetzt.
(b) die Chlorbuttersäure mit einem primären oder sekundären Alkohol mit 4 bis 8 Kohlenstoffatomen verestert,
(c) den Ester mit einem Alkalialkoholat eines primären Alkohols mit 1 bis 3 Kohlenstoffatomen zu einem Gemisch von Cyclopropancarbonsäureestern cyclisiert,
(d) das Cyclopropancarbonsäureester-Gemisch durch Umsetzung mit Ammoniak in Cyclopropancarbonsäureamid umwandelt und
(e) das Cyclopropancarbonsäureamid durch Hofmannschen Abbau in Cyclopropylamin überführt.

Die Stufe (c) dieses Verfahrens löst die Aufgabe, eine verbesserte Cyclisierungsreaktion bereitzustellen.

Das Gesamtverfahren ergibt Cyclopropylamin mit einer Reinheit von >98% in Ausbeuten von etwa 70%, bezogen auf Butyrolacton. In der Stufe (c) werden die Cyclopropancarbonsäureester in einer Ausbeute von ca. 90% erhalten, bezogen auf den Chlorbuttersäureester.

Die Stufe (a) wird z.B. so durchgeführt, daß Butyrolacton in an sich bekannter Weise bei einer Temperatur, die zweckmäßig 135 bis 140°C nicht überschreitet, unter einem Druck von bis zu 25 bar und in der Regel ohne Katalysator in einer zunächst stark exothermen Reaktion mit trockenem Chlorwasserstoff umgesetzt wird.

In der Stufe (b) wird die Chlorbuttersäure zweckmäßig als Rohprodukt. das als Nebenprodukt gamma-(gamma-Chlorbutyryl)buttersäure enthält. mit einem primären oder sekundären Alkohol mit 4 bis 8 Kohlenstoffatomen verestert. Diese Ester sind im Gegensatz zu den Estern niederen Alkohole gut herstellbar. Bevorzugte Alkohole sind primäre Alkanole, beispielsweise 1-Butanol, 1-Pentanol und 1-Hexanol. Weiterhin gut geeignet sind sekundäre Alkanole, wie 2-Butanol, 2- oder 3-Pentanol sowie 2- oder 3-Hexanol. Verfahren zur Veresterung von primären oder sekundären Alkoholen sind gut bekannt. Man arbeitet z.B. bei Temperaturen von 120 bis 140°C ohne Katalysator.

Die Stufen (a) und (b) lassen sich auch kombinieren, indem man in das vorgelegte Butyrolacton gleichzeitig Chlorwasserstoff und den Alkohol einführt.

Die Stufe (c) ist ein wesentlicher Teil des Gesamtverfahrens. In ihr wird der Chlorbuttersäureester aus der Stufe (b) cyclisiert. Dazu verwendet man ein Alkalialkoholat, vorteilhaft ein Natriumalkoholat, eines Alkohols, zweckmäßig eines Alkanols, mit 1 bis 3 Kohlenstoffatomen. Das bevorzugte Alkalialkoholat ist Natriummethylat, das in technischen Mengen gut zugänglich ist. Das Alkalialkoholat wird zweckmäßig als Lösung in dem entsprechenden Alkohol angewandt und zweckmäßig in Mengen von 1 bis 1,5 Äquivalenten je Äquivalent Chlorbuttersäureester eingesetzt. Der Ringschluß kann ohne Zusatz eines weiteren inerten Lösemittels bewirkt werden, indem man bei einer Temperatur, die vorteilhaft 80 bis 150°C beträgt, die Alkohol/Alkoholat-Lösung vorlegt, gegebenenfalls einen Teil des als Lösemittel für das Alkoholat dienenden Alkohols abdestilliert und den rohen Chlorbuttersäureester unter Rühren zudosiert. Als Lösemittel eingesetzten sowie durch Umesterung aus der Carbonester-Funktion entstehender Alkohol können noch während der Reaktion abdestilliert werden.

Das Reaktionsprodukt ist ein Gemisch von Cyclopropancarbonsäurestern, die die ursprüngliche Carbonester-Funktion sowie eine durch Umesterung entstandene Carbonester-Funktion mit einer Alkohol-Komponente enthalten, die aus dem als Alkohol verwendeten Lösemittel für das Alkoholat stammt. Das Gemisch kann ohne weitere Aufarbeitung in der Folgestufe verwendet werden.

In der Stufe (d) wird das Cyclopropancarbonsäureestergemisch der Stufe (c) zum Cyclopropancarbonsäureamid umsetzt. Das kann nach einem der im Stand der Technik beschriebenen Verfahren geschehen. Man geht beispielsweise so vor, daß man das Estergemisch bei einer Temperatur von 40 bis 120°C, vorzugsweise von 60 bis 80°C, mit Ammoniak bei einem Druck von 1,0 bis 5,0 bar umsetzt. Die Umsetzung verläuft erstaunlich glatt, obwohl das Edukt eine Estergemisch ist.

Auch hinsichtlich der Stufe (e) unterscheidet sich das Gesamtverfahren im Prinzip nicht von den entsprechenden Verfahren des Standes der Technik. So kann man eine Suspension oder eine Lösung des rohen Cyclopropancarbonsäureamids in Wasser, mit oder ohne vorherige Abtrennung restlicher organischer Lösemittel, dem Hofmannschen Abbau unterwerfen. Man wendet das Hypohalogenit, vorzugsweise Natriumhypochlorit, vorteilhaft in Mengen von 1,0 bis 1,5 Äquivalenten, insbesondere von 1,0 bis 1,2 Äquivalenten, und die Base, vorzugsweise Natriumhydroxid, vorteilhaft in Mengen von 1,5 bis 2,5 Äquivalenten, insbesondere von 1,8 bis 2,2 Äquivalenten an, jeweils bezogen auf die Carbonamid-Funktion, und arbeitet im allgemeinen bei Temperaturen von 40 bis 150 °C, insbesondere von 60 bis 80°C.

Die folgenden Beispiele sollen die Erfindung weiter erläutern, nicht aber den Schutzbereich begrenzen, wie er sich aus den Patentansprüchen ergibt.

### Beispiele

### Beispiel 1

Stufen (a) und (b): 86,1 g (1.0 mol) Butyrolacton werden unter Rühren auf eine Temperatur von 135°C erhitzt. Innerhalb von 4,5 h werden 80,0 g (1,07 mol) n-Butanol und ein Überschuß von gasförmigem Chlorwasserstoff zudosiert, wobei eine Reaktionstemperatur von 130 bis 135°C eingehalten wird. Während der Reaktion werden 124,0 g Destillat abgenommen. Das Reaktionsgemisch wird anschließend bei ca. 70°C und einem Druck von 60 mbar von überschüssigem n-Butanol befreit. Es verbleibt ein Rückstand von 211,2 g.
Stufe (c): Aus 225,0 g (1,25 mol) 30-gewichtsprozentiger methanolischer Natriummethylat-Lösung wird bis zu einer Sumpftemperatur von 100°C Methanol durch Destillation entfernt. Anschließend dosiert man innerhalb von 1,5 h den rohen Chlorbuttersäurebutylester aus den Stufen (a) und (b) hinzu. Dabei wird eine Temperatur im Reaktionsgemisch von 97 bis 100°C eingehalten, während man kontinuierlich ein Gemisch aus Methanol und n-Butanol abdestilliert. Nach beendeter Zudosierung wird noch für 2 h eine Sumpftemperatur von ca. 100°C gehalten.
Stufe (d): Das Reaktionsgemisch der Stufe (c) wird auf 60°C abgekühlt und bei Normaldruck mit einem überschuß an gasförmigem Ammoniak umgesetzt. Die Reaktionszeit beträgt ca. 10 h. Nach dem Abkühlen auf Raumtemperatur wird das Reaktionsgemisch mit 80,0 g 20-gewichtsprozentiger Salzsäure neutral gestellt. Lösemittelreste werden über eine Kolonne bis zu einer Sumpftemperatur von 120°C herausdestilliert. Man läßt das Gemisch abkühlen und versetzt es mit soviel Wasser, daß eine homogene Lösung (659,4 g) entsteht. Gemäß Bestimmung durch HPLC liegt das Cyclopropancarbonsäureamid in einer Ausbeute von 70 % vor, bezogen auf Butyrolacton.
Stufe (e): Zu der Lösung aus der Stufe (d) gibt man bei 0°C 160,0 g (2,0 mol) 50-gewichtsprozentige Natronlauge und erwärmt das Gemisch auf etwa 15°C. Anschließend gibt man bei dieser Temperatur 1010,9 g (1,0 mol) 7,3-gewichtsprozentige wäßrige Natriumhypochloritlösung hinzu und läßt das Gemisch bei dieser Temperatur 20 min rühren. Danach erwärmt man für 5 min auf 60°C. Das Cyclopropylamin wird als wäßrige Lösung durch Destillation über eine Kolonne gewonnen (163g). Mit Hilfe von GC und einer Säure/Base-Titration wird eine Cyclopropylaminausbeute von 66 % ermittelt, bezogen auf Butyrolacton.

### Beispiel 2

Stufen (a) und (b): 86,1 g (1,0 mol) Butyrolacton werden unter Rühren auf eine Temperatur von 135°C erhitzt. Innerhalb von 3 h werden 120,0 g (1,6 mol) n-Butanol und ein Überschuß von gasförmigem Chlorwasserstoff zudosiert, wobei eine Reaktionstemperatur von 134 bis 138°C eingehalten wird. Während der Reaktion werden 124 g Destillat abgenommen. Das Reaktionsgemisch wird anschließend bei ca. 70°C und einem Druck von 60 mbar von überschüssigem n-Butanol befreit. Es verbleibt ein Rückstand von 218,3 g.
Stufe (c): Aus 225,0 g (1,25 mol) 30-gewichtsprozentiger methanolischer Natriummethylat-Lösung wird bis zu einer Sumpftemperatur von 100°C Methanol durch Destillation entfernt. Anschließend dosiert man innerhalb von 1,5 h den rohen Chlorbuttersäurebutylester aus den Stufen (a) und (b) hinzu. Dabei wird eine Temperatur im Reaktionsgemisch von 97 bis 100°C eingehalten, während man kontinuierlich eine Gemisch aus Methanol und n-Butanol abdestilliert. Nach beendeter Zudosierung wird noch für 2 h eine Sumpftemperatur von ca. 100°C gehalten.
Stufe (d): Das Reaktionsgemisch der Stufe (c) wird auf 60°C abgekühlt und bei Normaldruck mit einem Überschuß an gasförmigem Ammoniak umgesetzt. Die Reaktionszeit beträgt ca. 5 h. Nach dem Abkühlen auf Raumtemperatur wird das Reaktionsgemisch mit 20-gewichtsprozentiger Salzsäure neutral gestellt. Lösemittelreste werden über eine Kolonne bis zu einer Sumpftemperatur von 120°C herausdestilliert. Man läßt das Gemisch abkühlen und versetzt es mit soviel Wasser, daß eine homogene Lösung (622,4 g) entsteht. Gemäß ßestimmung durch HPLC liegt das Cyclopropancarbonsäureamid in einer Ausbeute von 73 % vor, bezogen auf Butyrolacton.
Stufe (e): Zu der Lösung aus der Stufe (d) gibt man bei 0°C 160,0 g (2,0 mol) 50-gewichtsprozentige Natronlauge und erwärmt das Gemisch auf etwa 15°C. Anschließend gibt man bei dieser Temperatur 1010,9 g (1,0 mol) 7,3-gewichtsprozentige wäßrige Natriumhypochloritlösung hinzu und läßt das Gemisch bei dieser Temperatur 20 min rühren. Danach erwärmt man für 5 min auf 60°C. Das Cyclopropylamin wird als wäßrige Lösung durch Destillation über eine Kolonne gewonnen (177,0g). Mit Hilfe von GC und einer Säure/Base-Titration wird eine Cyclopropylaminausbeute von 69 % ermittelt, bezogen auf Butyrolacton.

## Patentansprüche

1. Verfahren zur Herstellung von Cyclopropylamin, ausgehend von Butyrolacton, dadurch gekennzeichnet, daß man
(a) Butyrolacton mit Chlorwasserstoff zu Chlorbuttersäure umsetzt,
(b) die Chlorbuttersäure mit einem primären oder sekundären Alkohol mit 4 bis 8 Kohlenstoffatomen verestert,
(c) den Ester mit einem Alkalialkoholat eines primären Alkohols mit 1 bis 3 Kohlenstoffatomen zu einem Gemisch von Cyclopropancarbonsäureestern cyclisiert,
(d) das Cyclopropancarbonsäureester-Gemisch durch Umsetzung mit Ammoniak in Cyclopropancarbonsäureamid umwandelt und
(e) das Cyclopropancarbonsäureamid durch Hofmannschen Abbau in Cyclopropylamin überführt.

2. Verfahren zur Herstellung von Cyclopropancarbonsäureestern, dadurch gekennzeichnet, daß man einen Chlorbuttersäureester eines primären, sekundären oder tertiären Alkohols mit einem Alkalialkoholat eines primären Alkohols mit 1 bis 3 Kohlenstoffatomen zum Cyclopropancarbonsäureester cyclisiert,

3. Verfahren nach einem der Ansprüche 1 oder 2, dadurch gekennzeichnet, daß man 1 bis 1,5 Äquivalente Alkalialkoholat je Äquivalent Chlorbuttersäureester anwendet.

4. Verfahren nach einem der Ansprüche 1 bis 3, dadurch gekennzeichnet, daß das Alkalialkoholat Natriummethylat ist.

5. Verfahren nach einem der Ansprüche 1 bis 4, dadurch gekennzeichnet, daß die Cyclisierungsreaktion bei einer Temperatur von 80 bis 150°C durchgeführt wird.

6. Verfahren nach einem der Ansprüche 1 bis 5, dadurch gekennzeichnet, daß das Reaktionsgemisch der Cyclisierungsreaktion ohne Aufarbeitung direkt mit Ammoniak zu Cyclopropancarbonsäureamid umgesetzt wird.
